# EUROPEAN PATENT APPLICATION

(11) **EP 4 227 673 A1**
(43) Date of publication of application: **16.08.2023**
(21) Application number: 23153391.0
(22) Date of filing: 26.01.2023
(51) Int. Cl.: G01N 21/84, G01N 21/89, G01N 21/898, G01B 11/04, G01B 11/245, G01N 33/46, G01N 21/88

(54) **APPARATUS FOR CAPTURING IMAGES OF ELONGATED TIMBER**

(30) Priority: 27.01.2022 FI 20221014
(71) Applicant: Finnos Oy, 53900 Lappeenranta (FI)
(72) Inventor: Toivanen, Toni, 53900 Lappeenranta (FI); Heikkinen, Jere, 53900 Lappeenranta (FI); Smagin, Jyri, 53900 Lappeenranta (FI); Söderström, Juha, 53900 Lappeenranta (FI); Alatalo, Juha, 53900 Lappeenranta (FI); Salo, Jaakko, 53900 Lappeenranta (FI); Forsström, Jari, 53900 Lappeenranta (FI); Eskola, Kalle, 53900 Lappeenranta (FI); Sampo, Jouni, 53900 Lappeenranta (FI); Kuvaja, Esa, 53900 Lappeenranta (FI)
(74) Representative: Primrose Oy

(57) **Abstract**

The invention relates to apparatus (1) for capturing images of elongated timber (100), The apparatus (1) comprises one or more mirrors (10, 20, 50, 70), a first camera (30), and a conveyor (40) which is arranged to convey the timber (100) transversely to a transfer direction (K). The first camera (30) is arranged relative to one or more mirror surface (11, 21, 51, 71) so that the first camera (30) capturing images of the timber (100) only indirectly via the one or more mirror surfaces (11, 21, 51, 71).

## Description

### FIELD OF THE INVENTION

The present invention relates to apparatus for capturing images of elongated timber and more particularly to apparatus for capturing images of elongated timber according to preamble of the independent claim 1.

### BACKGROUND OF THE INVENTION

The purpose of capturing images of timber is to ensure the quality of the timber. Captured images are used to find out for example dimensions of each timber and possible defects at the edges of the timber. Furthermore, it is possible to find out knots, resin pockets, cracks, rots, blue-stains, and other aspects determining the quality and value of timber.

In the prior art, it is known to inspect timber with at least three cameras when the timber moves transversely to the cameras.

One of the problems associated with the prior art is the great number of cameras.

In the prior art, it is also known to inspect timber when the timber moves in a longitudinal direction with respect to the cameras.

One of the problems associated with the prior art is that capturing images is slow. Transferring timber in a longitudinal direction with respect to the cameras takes longer than transferring transversely as a length of timber is typically remarkably greater than a width.

In the prior art, it is also known to inspect timber when the timber moves transversely to the cameras so that the camera points both directly at the timber and at one or more mirrors.

One of the problems associated with the prior art is that positioning the camera requires large open space above the timber.

### BRIEF DESCRIPTION OF THE INVENTION

An object of the present invention is to solve or at least alleviate the disadvantages of prior art. Particularly, an object of the present invention is to provide apparatus for capturing images of elongated timber so that positioning of camera has many alternatives.

The objects of the invention are achieved by apparatus for capturing images of elongated timber which are characterized by what is stated in the independent claim 1.

The preferred embodiments of the invention are disclosed in the dependent claims.

Apparatus for capturing images of elongated timber according to the present invention comprises one or more mirrors having one or more mirror surfaces, a first camera, and a conveyor which is arranged to convey the timber transversely to a transfer direction. The first camera is arranged relative to one or more mirror surfaces so that the first camera is arranged to capture images of the timber only indirectly via the one or more mirror surfaces.

In one embodiment, the conveyor comprises a conveyor upper surface which is arranged to receive timber.

In one embodiment, the one or more mirrors comprise a first mirror and a second mirror, the one or more mirror surfaces comprise a first mirror surface and a second mirror surface so that the first mirror comprises the first mirror surface and the second mirror comprises the second mirror surface, the second mirror surface being transverse to the first mirror surface and transverse to the conveyor upper surface, and the second mirror surface is arranged to reflect a mirror image of the timber via the first mirror surface to the first camera.

The second mirror surface enables capturing images of timber from many directions.

In one embodiment, the conveyor comprises a conveyor upper surface which is arranged to receive timber; the one or more mirrors comprise a first mirror and a second mirror; the one or more mirror surfaces comprise a first mirror surface and a second mirror surface so that the first mirror comprises the first mirror surface and the second mirror comprises the second mirror surface; the second mirror surface being transverse to the first mirror surface and transverse to the conveyor upper surface, the first mirror surface is arranged to reflect a mirror image of the timber to the first camera, the second mirror surface is arranged to reflect a mirror image of the timber to the first camera, and the apparatus further comprises a second camera aimed at the conveyor upper surface.

This enables capturing images of timber from many directions.

In another embodiment, the conveyor comprises a conveyor upper surface which is arranged to receive timber, the one or more mirrors comprise a first mirror and a second mirror; the one or more mirror surfaces comprise a first mirror surface and a second mirror surface so that the first mirror comprises the first mirror surface and the second mirror comprises the second mirror surface, the second mirror surface being transverse to the first mirror surface and transverse to the conveyor upper surface, and the second mirror surface is arranged to reflect a mirror image of the timber via the first mirror surface to the first camera, the first mirror surface being at a first distance from the conveyor upper surface, the second mirror surface being at a second distance from the conveyor upper surface, and the first distance being unequal to the second distance.

This enables positioning cameras both in the vertical direction and in the transfer direction in different positions.

In third embodiment, the conveyor comprises a conveyor upper surface which is arranged to receive timber, the one or more mirrors comprise a first mirror and a second mirror, the one or more mirror surfaces comprise a first mirror surface and a second mirror surface so that the first mirror comprises the first mirror surface and the second mirror comprises the second mirror surface, the second mirror surface being transverse to the first mirror surface and transverse to the conveyor upper surface, and the second mirror surface is arranged to reflect a mirror image of the timber via the first mirror surface to the first camera, the first mirror surface being at a first distance from the conveyor upper surface, the second mirror surface being at a second distance from the conveyor upper surface, and the first distance being greater than the second distance.

This enables positioning cameras both in the vertical direction and in the transfer direction in different positions so that camera is closer to the conveyor upper surface than the first mirror surface.

In one embodiment, the one or more mirrors comprise a first mirror, a second mirror and a third mirror, the one or more mirror surfaces comprise a first mirror surface, a second mirror surface and a third mirror surface so that the first mirror comprises the first mirror surface, the second mirror comprises the second mirror surface and the third mirror comprises a third mirror surface.

The third mirror surface enables capturing images of timber in more directions.

In an alternative embodiment, the one or more mirrors comprise a first mirror, a second mirror and a third mirror, the one or more mirror surfaces comprise a first mirror surface, a second mirror surface and a third mirror surface so that the first mirror comprises the first mirror surface, the second mirror comprises the second mirror surface and the third mirror comprises a third mirror surface, the third mirror surface is positioned transverse to the first mirror surface and transverse to the conveyor upper surface, the third mirror surface facing in a different direction than the second mirror surface, and the third mirror surface is arranged to reflect a mirror image of the timber via the first mirror surface to the first camera.

This enables capturing images of timber from several directions and increases options for positioning cameras.

In an another alternative embodiment, the one or more mirrors comprise a first mirror, a second mirror and a third mirror, the one or more mirror surfaces comprise a first mirror surface, a second mirror surface and a third mirror surface so that the first mirror comprises the first mirror surface, the second mirror comprises the second mirror surface and the third mirror comprises a third mirror surface, the third mirror surface is positioned transverse to the first mirror surface and transverse to the conveyor upper surface, the third mirror surface facing in a different direction than the second mirror surface, and the third mirror surface is arranged to reflect a mirror image of the timber via the first mirror surface to the first camera, and the third mirror is arranged at a distance from the second mirror in the transfer direction.

The third mirror surface enables capturing images of timber from even more directions.

In a yet another alternative embodiment, the one or more mirrors comprise a first mirror, a second mirror and a third mirror, the one or more mirror surfaces comprise a first mirror surface, a second mirror surface and a third mirror surface so that the first mirror comprises the first mirror surface, the second mirror comprises the second mirror surface and the third mirror comprises a third mirror surface, the third mirror surface is positioned transverse to the first mirror surface and transverse to the second mirror surface, the first mirror surface, the second mirror surface and the third mirror surface are arranged to reflect mirror images of the timber from different directions to the first camera.

The third mirror surface enables capturing images of timber from even more directions.

In a yet another alternative embodiment, the one or more mirrors comprise a first mirror, a second mirror and a third mirror, the one or more mirror surfaces comprise a first mirror surface, a second mirror surface and a third mirror surface so that the first mirror comprises the first mirror surface, the second mirror comprises the second mirror surface and the third mirror comprises a third mirror surface, the third mirror surface is positioned transverse to the first mirror surface and transverse to the conveyor upper surface, the third mirror surface facing away from the second mirror surface, the second mirror surface facing away from the third mirror surface, third mirror surface is arranged to reflect a mirror image of the timber via the first mirror surface to the first camera, and the third mirror is arranged at a distance from the second mirror in the transfer direction.

The third mirror surface enables capturing images of timber from even more directions and this position of the third mirror increases options for positioning the camera.

In a yet another alternative embodiment, the one or more mirrors comprise a first mirror, a second mirror and a third mirror, the one or more mirror surfaces comprise a first mirror surface, a second mirror surface and a third mirror surface so that the first mirror comprises the first mirror surface, the second mirror comprises the second mirror surface and the third mirror comprises a third mirror surface, the third mirror surface is positioned transverse to the first mirror surface and transverse to the conveyor upper surface, the third mirror surface facing towards the second mirror surface, the second mirror surface facing towards the third mirror surface, third mirror surface is arranged to reflect a mirror image of the timber via the first mirror surface to the first camera, and the third mirror is arranged at a distance from the second mirror in the transfer direction.

The third mirror surface enables capturing images of timber from even more directions and this position of the third mirror increases options for positioning the camera.

In one embodiment, a distance between the first camera and the conveyor upper surface being less than a distance between the first mirror surface and the conveyor upper surface.

Thus, positioning the mirror and the camera requires less space in the vertical direction.

In a yet another alternative embodiment, a distance between the first camera and the conveyor upper surface being less than a distance between the first mirror surface and the conveyor upper surface, and the first mirror surface being parallel to the conveyor upper surface.

Thus, positioning the mirror and the camera requires less space in the vertical direction and image on the first mirror being parallel to the timber.

In a yet third alternative embodiment, distance between the first camera and the conveyor upper surface being greater than a distance between the first mirror surface and the conveyor upper surface.

Thus, the camera may be positioned in the transfer direction in a desired position.

In a yet fourth alternative embodiment, the one or more mirrors comprise a first mirror, a second mirror, a third mirror and a fourth mirror; the one or more mirror surfaces comprise a first mirror surface, a second mirror surface, a third mirror surface and a fourth mirror surface so that the first mirror comprises the first mirror surface, the second mirror comprises the second mirror surface, the third mirror comprises a third mirror surface and the fourth mirror comprises the fourth mirror surface, and the fourth mirror surface is arranged to reflect at least a part of the first mirror surface to the first camera.

Thus, the camera may be positioned in the transfer direction in a desirable position and at the desired height.

In a yet fifth alternative embodiment, the one or more mirrors comprise a first mirror, a second mirror, a third mirror and a fourth mirror, the one or more mirror surfaces comprise a first mirror surface, a second mirror surface, a third mirror surface and a fourth mirror surface so that the first mirror comprises the first mirror surface, the second mirror comprises the second mirror surface, the third mirror comprises a third mirror surface and the fourth mirror comprises the fourth mirror surface, a distance between the fourth mirror surface and the conveyor upper surface being less than a distance between the first mirror surface and the conveyor upper surface, and the fourth mirror surface is arranged to reflect at least a part of the first mirror surface to the first camera.

Thus, the camera may be positioned in the transfer direction in a desired position and at the desired height.

In one alternative embodiment, the first camera comprises a matrix camera.

Matrix camera provides a suitable image of the timber.

In a yet another alternative embodiment, the apparatus comprises a second camera aimed at the conveyor upper surface, the first camera comprises a matrix camera and the second camera comprises a matrix camera.

The second camera may be positioned close to the timber.

In a yet third alternative embodiment, the first camera comprises a matrix camera and the first mirror surface is arranged facing towards the conveyor upper surface.

This enables positioning the first camera close to the timber of which images will be captured.

In a yet fourth alternative embodiment, the first camera comprises a matrix camera, and the first mirror surface comprises a first surface and a second surface so that the first surface being transverse to the second surface.

This enables positioning the first camera close to the timber of which images will be captured and capturing images from several directions.

In a yet fifth alternative embodiment, the first camera comprises a matrix camera, and the first mirror surface comprises a first surface, a second surface and a third surface so that the first surface, the second surface and the third surface being transverse to each other.

This enables positioning the first camera close to the timber of which images will be captured, and capturing images from even more directions.

In one embodiment, the first camera is arranged above the conveyor upper surface.

In an alternative embodiment, the first camera is arranged below the conveyor.

In another alternative embodiment, the first camera is arranged above the conveyor upper surface and the second camera is arranged below the conveyor upper surface.

In a yet another alternative embodiment, the first camera is arranged below the conveyor upper surface and the second camera is arranged above the conveyor upper surface.

In a yet another alternative embodiment, the first camera and the second camera are arranged on different sides of the conveyor upper surface.
the first camera and the second camera on different sides of the conveyor upper surface enable capturing images of an upper surface and a lower surface of the timber simultaneously.

An advantage of the apparatus for capturing images according to the invention is that it enables capturing images of elongated timber from several directions with less cameras, so that the cameras may be placed at the desired distance from the timber of which images will be captured in the height direction, and in the transfer direction of the timber at desired point of the apparatus, and at the same time conveying the timber transversely relative to the camera in a transfer direction makes the image capturing faster.

### BRIEF DESCRIPTION OF THE DRAWINGS

The invention is described in detail by means of specific embodiments with reference to the enclosed drawings, in which
Figures 1 - 9 show schematically side views of the apparatus for capturing images of elongated timber according to different embodiments of the present invention; and
Figures 10 and 11 show schematically top views of the apparatus for capturing images of elongated timber according to different embodiments of the present invention.

### DETAILED DESCRIPTION OF THE INVENTION

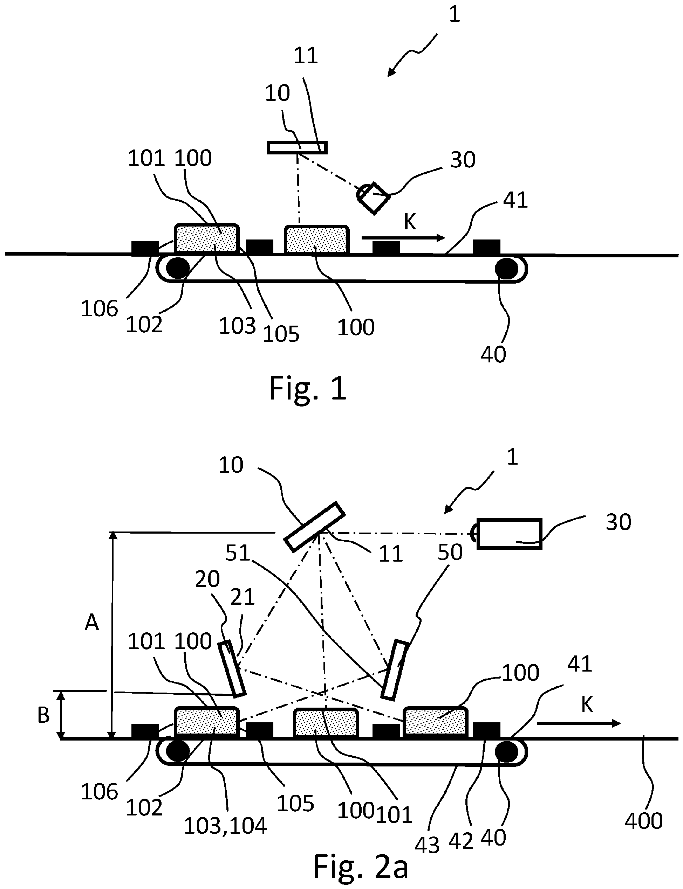

Figure 1 shows schematically side view of an apparatus for capturing images of elongated timber.

The apparatus 1 is provided for capturing images of elongated timber 100. Each elongated timber 100 comprises an upper surface 101, a lower surface 102, a first end surface 103, a second end surface 104, a first side surface 105 extending between the first end surface 103 and the second end surface 104 and a second side surface 106 extending between the first end surface 103 and the second end surface 104. The apparatus 1 comprises one or more mirrors 10, 20, 50, 70 which comprise one or more mirror surfaces 11, 21, 51, 71. The apparatus 1 further comprises a first camera 30. The apparatus 1 further comprises a conveyor 40. The first camera 30 is arranged relative to one or more mirror surfaces 11, 21, 51, 71 so that the first camera 30 is capturing images of the timber 100 only indirectly via the one or more mirror surfaces 11, 21, 51, 71.

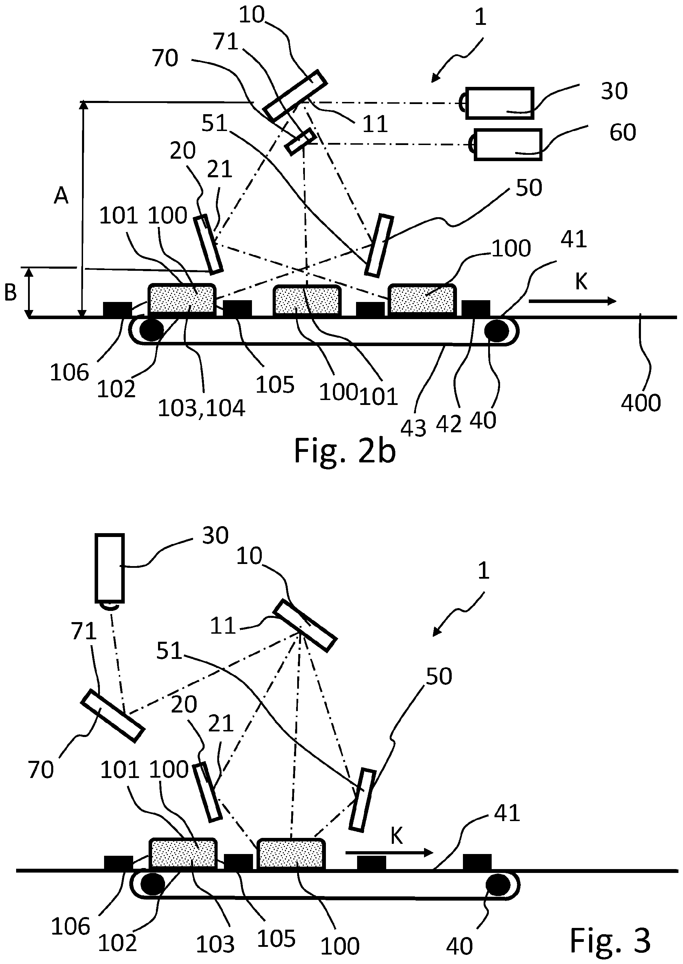

In the embodiment shown in figure 1, one or more mirrors 10, 20, 50, 70 comprise a first mirror 10. One or more mirror surfaces 11, 21, 51, 71 comprise a first mirror surface 11. For the sake of clarity, it is noted that in figure 3 is shown embodiments in which one or more mirrors 10, 20, 50, 70 comprise a first mirror 10, a second mirror 20, a third mirror 30 and a fourth mirror 70. In figure 3 is shown also embodiments in which one or more mirror surfaces 11, 21, 51, 71 comprise a first mirror surface 11, a second mirror surface 21, a third mirror surface 51 and a fourth mirror surface 71.

The conveyor 40 is arranged to convey the timber 100 transversely to a transfer direction K. The conveyor 40 comprises a conveyor upper surface 41 which is arranged to receive timber 100.

In one exemplary embodiment of the invention, the apparatus 1 comprises a main conveyor 400 arranged to convey timber 100 to the conveyer 40 and from the conveyer 40.

In the context of this application the conveyor upper surface 41 which is arranged to receive timber 100 means that the timber 100 is supported on the conveyor upper surface 41.

The conveyor 40 is arranged to move the timber in a transfer direction K.

In one exemplary embodiment of the invention, the conveyor 40 comprises one or more rake 42 and one or more chain of rake 43 which are arranged to transfer timber 100 on the conveyor upper surface 41.

In the context of this application, a mirror surface means a reflective surface that forms an image.

In one exemplary embodiment of the invention, the first camera 30 is arranged relative to one or more mirror surfaces 11, 21, 51, 71 so that the first camera 30 is arranged to capture images of the timber 100 only indirectly via the one or more mirror surfaces 11, 21, 51, 71, and the first camera 30 is arranged to capture images of the timber 100 on the conveyor upper surface 41.

The timber 100 having a longitudinal direction P between the first end surface 103 and the second end surface 104. In the context of this application, position of the timber 100 transversely to a transfer direction K means that the longitudinal direction P of the timber 100 is transverse to the transfer direction K. In other words, timber 100 is arranged to proceed such that the upper surface 101, the lower surface 102, the first side surface 105 or the second side surface 106 leading. In other words, the timber 100 is arranged to proceed transversely relative to the first camera 30.

In one preferred embodiment, the timber 100 is arranged to proceed the first side surface 105 or the second side surface 106 leading.

In one exemplary embodiment of the invention, a distance between the first camera 30 and the conveyor upper surface 41 being greater than a distance between the first mirror surface 11 and the conveyor upper surface 41.

In the context of this application, a distance being greater from the conveyor upper surface 41 means a position which is farther away upwards or downwards from the conveyor upper surface 41 when the apparatus is operable.

In the context of this application, a distance being less from the conveyor upper surface 41 means a position which is closer upwards or downwards from the conveyor upper surface 41 when the apparatus is operable.

In one exemplary embodiment of the invention, the first mirror surface 11 is parallel relative to the conveyor upper surface 41.

In an alternative embodiment, the first mirror surface 11 is transverse to the conveyor upper surface 41.

In one exemplary embodiment of the invention, the first camera 30 comprises a matrix camera.

In one exemplary embodiment of the invention, a mirror image formed on the first mirror surface 11 comprises at least a portion of the upper surface 101 of the timber 100.

Figure 2a shows schematically an apparatus for capturing images of elongated timber.

In one exemplary embodiment of the invention, the one or more mirrors 10, 20, 50, 70 comprise a first mirror 10 and a second mirror 20. The one or more mirror surfaces 11, 21, 51, 71 comprise a first mirror surface 11 and a second mirror surface 21 so that the first mirror 10 comprises the first mirror surface 11 and the second mirror 20 comprises the second mirror surface 21.

In this embodiment, the apparatus 1 further comprises the first camera 30 and the conveyor 40.

The conveyor 40 which is arranged to convey the timber 100 transversely to a transfer direction K. The conveyor 40 comprises the conveyor upper surface 41 which is arranged to receive timber 100. The second mirror surface 21 being transverse to the first mirror surface 11 and transverse to the conveyor upper surface 41. The second mirror surface 21 is arranged to reflect a mirror image of the timber 100 to the first camera 30 via the first mirror surface 11.

In one exemplary embodiment of the invention, the second mirror surface 21 is above the conveyor upper surface 41.

In one exemplary embodiment of the invention, the second mirror surface 21 is lower than the conveyor upper surface 41. In other words, the second mirror surface 21 is below the conveyor upper surface 41.

In one exemplary embodiment of the invention, the second mirror surface 21 is transverse to the transfer direction K. In other words, the second mirror surface 21 is positioned transversely relative to the first mirror surface 11, transversely relative to the conveyor upper surface 41 and relative to the transfer direction K.

In one embodiment, the first mirror surface 11 is at a first distance A from the conveyor upper surface 41, the second mirror surface 21 is at a second distance B from the conveyor upper surface 41, and the first distance A is unequal to the second distance B.

The first distance A and the second distance B mean the distances upwards from the conveyor upper surface 41 when the conveyor 40 is in a position where it is used for conveying timber.

In the context of this application, the first distance A means the shortest distance of the first mirror surface 11 in the vertical direction from the conveyor upper surface 41. In other words, the first distance A means the distance of the point of the first mirror surface 11 closest to the conveyor upper surface 41 from the conveyor upper surface 41.

In the context of this application, the second distance B means the shortest distance of the second mirror surface 21 in a vertical direction from the conveyor upper surface 41. In other words, the second distance B means the distance of the point of the second mirror surface 21 closest to the conveyor upper surface 41 from the conveyor upper surface 41.

In one embodiment, the first mirror surface 11 being at a first distance A from the conveyor upper surface 41, the second mirror surface 21 being at a second distance B from the conveyor upper surface 41, and the first distance A being greater than the second distance B.

In one exemplary embodiment of the invention, the one or more mirrors 10, 20, 50, 70 comprise a first mirror 10, a second mirror 20 and a third mirror 50. The one or more mirror surfaces 11, 21, 51, 71 comprise a first mirror surface 11, a second mirror surface 21 and a third mirror surface 51 so that the first mirror 10 comprises the first mirror surface 11, the second mirror 20 comprises the second mirror surface 21 and the third mirror 50 comprises a third mirror surface 51. The third mirror surface 51 is positioned transverse to the first mirror surface 11 and transverse to the conveyor upper surface 41. The third mirror surface 51 is arranged to reflect a mirror image of the timber 100 via the first mirror surface 11 to the first camera 30. The third mirror surface 51 facing in a different direction than the second mirror surface 21.

The third mirror surface 51 facing in a different direction than the second mirror surface 21 means that the third mirror surface 51 and the second mirror surface 21 are not parallel to each other and they do not face in the same direction.

In one exemplary embodiment of the invention, the third mirror surface 51 is transverse to the transfer direction K.

In one exemplary embodiment of the invention, the third mirror 50 is arranged in the transfer direction K at a distance from the second mirror 20.

In one exemplary embodiment of the invention, the third mirror surface 51 faces away from the second mirror surface 21 and the second mirror surface 21 faces away from the third mirror surface 51.

In an alternative exemplary embodiment of the invention, the third mirror surface 51 faces towards the second mirror surface 21 and the second mirror surface 21 faces towards the third mirror surface 51.

In one exemplary embodiment of the invention, the first mirror surface 11 is parallel to the conveyor upper surface 41.

In one exemplary embodiment of the invention, the first camera 30 comprises a matrix camera.

In one exemplary embodiment of the invention, the first camera 30 comprises a line-scan camera.

In one exemplary embodiment of the invention, the first camera 30 comprises a CCD sensor or a CMOS sensor.

In one exemplary embodiment of the invention, the first camera 30 comprises a lens.

In one exemplary embodiment of the invention, the mirror image formed on the first mirror surface 11 comprises at least a portion of the upper surface 101 of the timber 100.

The embodiments shown in figure 2a may be combined with the embodiments shown in figure 1.

Figure 2b shows schematically an apparatus for capturing images of elongated timber.

In one exemplary embodiment of the invention, the one or more mirrors 10, 20, 50, 70 comprise a first mirror 10 and a second mirror 20. The one or more mirror surfaces 11, 21, 51, 71 comprise a first mirror surface 11 and a second mirror surface 21 so that the first mirror 10 comprises the first mirror surface 11 and the second mirror 20 comprises the second mirror surface 21.

In this embodiment, the apparatus 1 further comprises a first camera 30, a second camera 60 and a conveyor 40.

In one exemplary embodiment of the invention, the one or more mirrors 10, 20, 50, 70 comprise a first mirror 10, a second mirror 20 and a third mirror 50. The one or more mirror surfaces 11, 21, 51, 71 comprise a first mirror surface 11, a second mirror surface 21 and a third mirror surface 51 so that the first mirror 10 comprises the first mirror surface 11, the second mirror 20 comprises the second mirror surface 21 and the third mirror 50 comprises a third mirror surface 51. The third mirror surface 51 is positioned transverse to the first mirror surface 11 and transverse to the conveyor upper surface 41. The third mirror surface 51 is arranged to reflect a mirror image of the timber 100 via the first mirror surface 11 to the first camera 30. The third mirror surface 51 facing in a different direction than the second mirror surface 21.

The third mirror surface 51 facing in a different direction than the second mirror surface 21 means that the third mirror surface 51 and the second mirror surface 21 are not parallel to each other and they do not face in same direction.

In one exemplary embodiment of the invention, the one or more mirrors 10, 20, 50, 70 further comprise a fourth mirror 70. The one or more mirror surfaces 11, 21, 51, 71 comprise a fourth mirror surface 71 so that the fourth mirror 70 comprises the fourth mirror surface 71.

In one exemplary embodiment of the invention, the mirror image formed on the fourth mirror surface 71 comprises at least a portion of the upper surface 101 of the timber 100

In one exemplary embodiment of the invention, the mirror image formed on the fourth mirror surface 71 comprises at least a portion of the timber 100.

In one exemplary embodiment of the invention, the fourth mirror surface 71 is arranged to reflect at least a portion of the timber 100 to the second camera 60.

In one exemplary embodiment of the invention, the first camera 30 captures images of the timber 100 only indirectly via the one or more mirrors 10, 20, 50, 70 or via the one or more mirror surfaces 11, 21, 51, 71 and the second camera 30 captures images of the timber 100 only indirectly via the one or more mirrors 10, 20, 50, 70 or via one or more mirror surfaces 11, 21, 51, 71.

The embodiments shown in figure 2b may be combined with the embodiments shown in figures 1 and 2a.

Figure 3 shows schematically an apparatus for capturing images of elongated timber.

In one exemplary embodiment of the invention, the one or more mirrors 10,20, 50, 70 comprise a first mirror 10, a second mirror 20, a third mirror 50 and a fourth mirror 70; the one or more mirror surfaces 11, 21, 51, 71 comprise a first mirror surface 11, a second mirror surface 21, a third mirror surface 51 and a fourth mirror surface 71 so that the first mirror 10 comprises the first mirror surface 11, the second mirror 20 comprises the second mirror surface 21, the third mirror 50 comprises a third mirror surface 51 and the fourth mirror 70 comprises the fourth mirror surface 71, and the fourth mirror surface 71 is arranged to reflect at least a part of the first mirror surface 11 to the first camera 30.

In one exemplary embodiment of the invention, the fourth mirror surface 71 is closer to the conveyor upper surface 41 than the first mirror surface 11, and the fourth mirror surface 71 is arranged to reflect at least a part of the first mirror surface 11 to the first camera 30. In one exemplary embodiment of the invention, the first camera 30 is arranged relative to the first mirror surface 11, the second mirror surface 21, the third mirror surface 51 and the fourth mirror surface 71 so that the first camera 30 captures images of the timber 100 only indirectly on the fourth reflective mirror 71, the fourth mirror surface 71 is arranged to reflect at least a part of the first mirror surface 11 to the first camera 30 and the second mirror surface 21 and the third mirror surface 51 are arranged to face towards the timber 100 and the second mirror surface 21 and the third mirror surface 51 are arranged to reflect on the first mirror surface 11.

In one exemplary embodiment of the invention, the first camera 30 is arranged relative to the first mirror surface 11, the second mirror surface 21, the third mirror surface 51 and the fourth mirror surface 71 so that the first camera 30 captures images of the timber 100 only indirectly on the fourth reflective mirror 71, the fourth mirror surface 71 is arranged to reflect at least a part of the first mirror surface 11 to the first camera 30 and the second mirror surface 21 is arranged to face towards the timber 100 and the second mirror surface 21 is arranged to reflect on the first mirror surface 11.

The embodiments shown in figure 3 may be combined with the embodiments shown in figures 1, 2a and 2b.

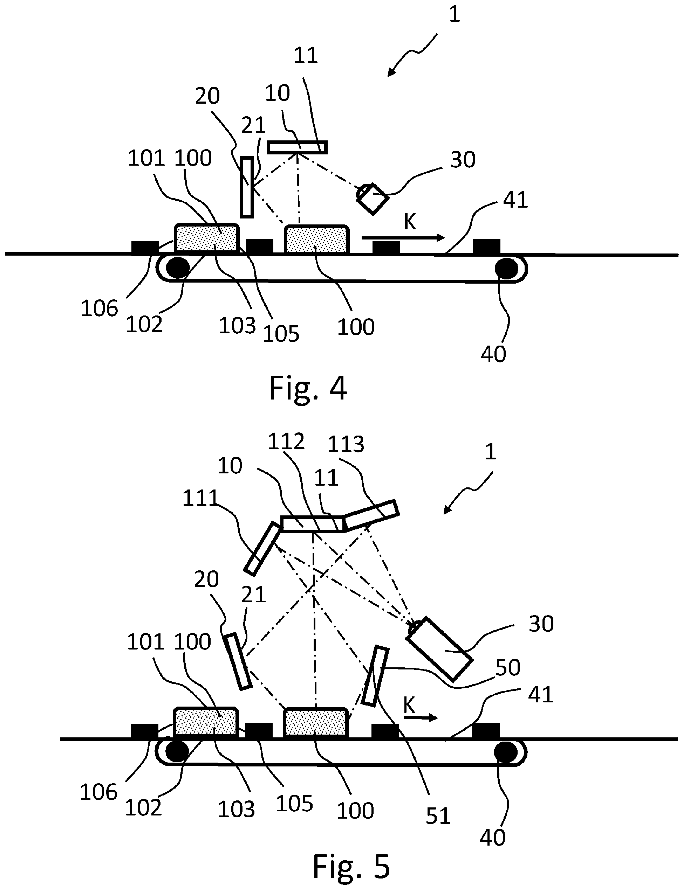

Figure 4 shows schematically an apparatus for capturing images of elongated timber.

In one exemplary embodiment of the invention, the first mirror surface 11 and the second mirror surface 21 are positioned transversely to each other.

In one exemplary embodiment of the invention, the first mirror surface 11 and the second mirror surface 21 are positioned perpendicular to each other.

The embodiments shown in figure 4 may be combined with the embodiments shown in figures 1, 2a, 2b and 3.

Figure 5 shows schematically an apparatus for capturing images of elongated timber.

In one exemplary embodiment of the invention, the first camera 30 comprises a matrix camera, and the first mirror surface 11 comprises a first surface 111 and a second surface 112 so that the first surface 111 is transverse to the second surface 112.

In one exemplary embodiment of the invention, the first camera 30 comprises a matrix camera, and the first mirror surface 11 comprises a first surface 111, a second surface 112 and a third surface 113 so that the first surface 111, the second surface 112 is transverse to the third surface 113.

The first surface 111 and the second surface 112 may be in contact with each other or there may be a gap between the first surface 111 and the second surface 112.

The first surface 111 and the third surface 113 may be in contact with each other or there may be a gap between the first surface 111 and the third surface 113.

In one exemplary embodiment of the invention, the first surface 111 and the second surface 112 facing towards the conveyor upper surface 41.

In one exemplary embodiment of the invention, the first surface 111, the second surface 112 and the third surface 113 face towards the conveyor upper surface 41.

The embodiments shown in figure 5 may be combined with the embodiments shown in figures 1, 2a, 2b, 3 and 4.

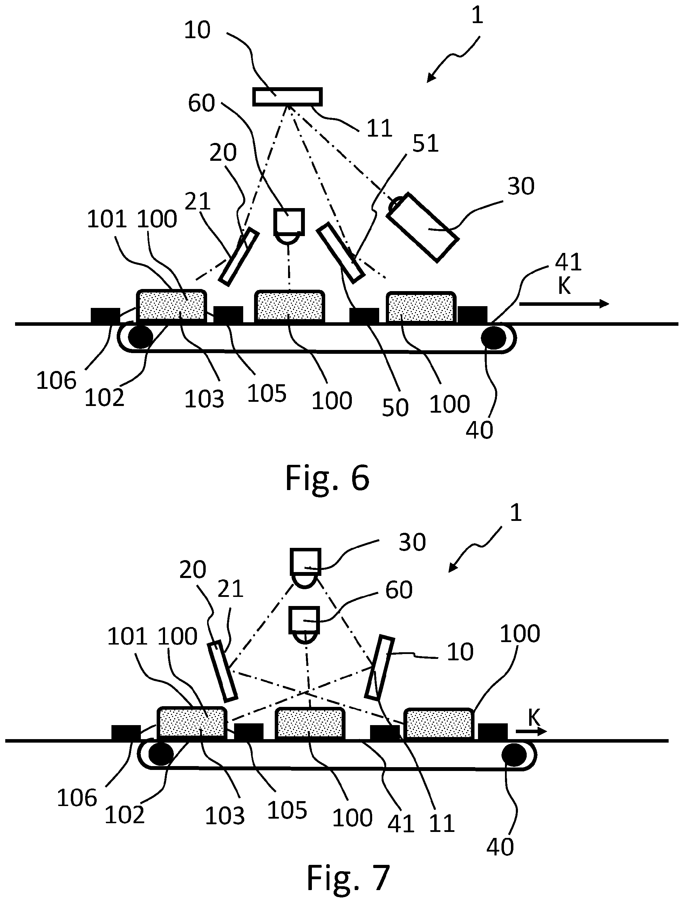

Figure 6 shows schematically an apparatus for capturing images of elongated timber.

In one exemplary embodiment of the invention, the apparatus 1 comprises a second camera 60 aimed at the conveyor upper surface 41.

In other words, the second camera 60 directly captures images of the timber 100.

In one exemplary embodiment of the invention, the second camera 60 comprises a matrix camera.

In one exemplary embodiment of the invention, the second camera 60 comprises a CCD sensor or a CMOS sensor.

In one exemplary embodiment of the invention, the second camera 60 comprises a line-scan camera.

The second camera may be positioned for instance beside the second mirror 20 or beside the first mirror 10 or between the second mirror 20 and the third mirror 50.

In one exemplary embodiment of the invention, the second camera 60 comprises a lens.

The embodiments shown in figure 6 may be combined with the embodiments shown in figures 1, 2a, 2b, 3, 4 and 5.

Figure 7 shows schematically an apparatus for capturing images of elongated timber.

In one exemplary embodiment of the invention, the conveyor 40 comprises a conveyor upper surface 41 which is arranged to receive timber 100. The one or more mirrors 10, 20, 50, 70 comprise a first mirror 10 and a second mirror 20. The one or more mirror surfaces 11, 21, 51, 71 comprise a first mirror surface 11 and a second mirror surface 21 so that the first mirror 10 comprises the first mirror surface 11 and the second mirror 20 comprises the second mirror surface 21. The second mirror surface 21 being transverse to the first mirror surface 11 and transverse to the conveyor upper surface 41, the first mirror surface 11 is arranged to reflect a mirror image of the timber 100 to the first camera 30, the second mirror surface 12 is arranged to reflect a mirror image of the timber 100 to the first camera 30. The apparatus 1 further comprises a second camera 60 aimed at the conveyor upper surface 41.

In one exemplary embodiment of the invention, the second camera 60 is arranged between the first camera 30 and the conveyor upper surface 41. In other words, the second camera 60 is closer to the conveyor upper surface 41 than the first camera 30.

The embodiments shown in figure 7 may be combined with the embodiments shown in figures 1, 2a, 2b, 3, 4, 5 and 6.

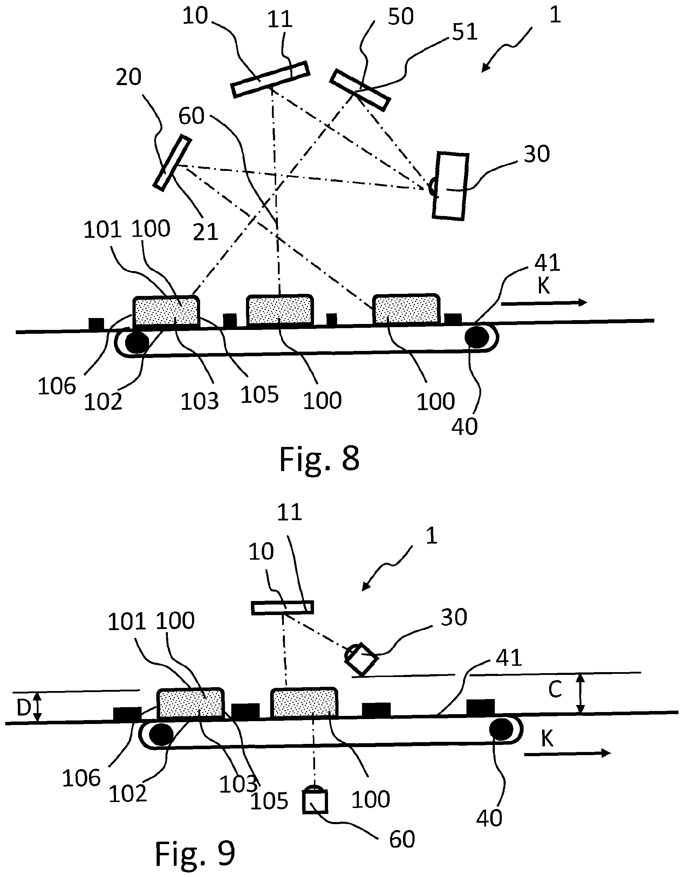

Figure 8 shows schematically an apparatus for capturing images of elongated timber.

In this embodiment, the one or more mirrors 10, 20, 50, 70 comprise a first mirror 10, a second mirror 20 and a third mirror 50. The one or more mirror surfaces 11, 21, 51, 71 comprise a first mirror surface 11, a second mirror surface 21 and a third mirror surface 51, so that the first mirror 10 comprises the first mirror surface 11, the second mirror 20 comprises the second mirror surface 21, and the third mirror 50 comprises the third mirror surface 51. The third mirror surface 51 is positioned transverse to the first mirror surface 11 and transverse to the second mirror surface 21. The first mirror surface 11, the second mirror surface 21 and the third mirror surface 51 are arranged to reflect mirror images of the timber 100 from different directions to the first camera 30.

The embodiments shown in figure 8 may be combined with the embodiments shown in figures 1, 2a, 2b, 3, 4, 5, 6 and 7.

Figure 9 shows schematically an apparatus for capturing images of elongated timber.

In one exemplary embodiment of the invention, the first camera 30 is arranged above the conveyor upper surface 41.

In an alternative exemplary embodiment of the invention, the first camera 30 is arranged below the conveyor upper surface 41 (not shown in the figure).

In one exemplary embodiment of the invention, the first camera 30 is arranged above the conveyor upper surface 41 and the second camera 60 is arranged below the conveyor upper surface 41.

In an alternative exemplary embodiment of the invention, the first camera 30 is arranged below the conveyor upper surface 41 and the second camera 60 is arranged above the conveyor upper surface 41 (not shown in the figure).

In one exemplary embodiment of the invention one or more mirrors 10, 20, 50, 70 are arranged above the conveyor upper surface 41.

In an alternative exemplary embodiment, the one or more mirrors 10, 20, 50, 70 are arranged below the conveyor upper surface 41 (not shown in the figure).

In one exemplary embodiment of the invention, the apparatus 1 having a clearance C, which is the shortest distance of the one more mirrors 10, 20, 50, 70, the first camera 30 and the second camera 60 upwards from the conveyor upper surface 41, the timber 100 having a thickness D from the conveyor upper surface 41 to the timber upper surface 101 and the clearance C is greater than or equal to the thickness D of the timber 100.

The embodiments shown in figure 9 may be combined with the embodiments shown in figures 1, 2a, 2b, 3, 4, 5, 6, 7 and 8.

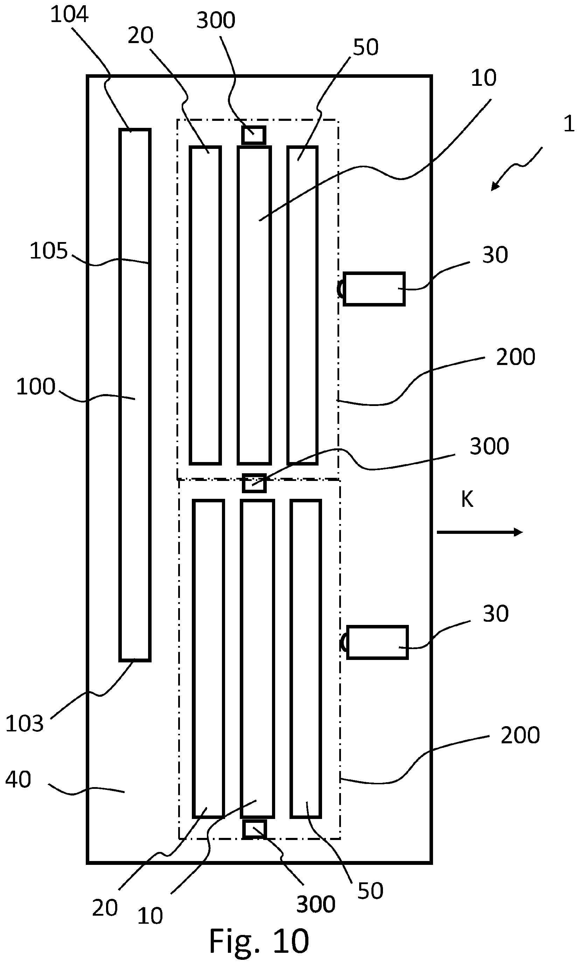

Figure 10 shows schematically a top view of an apparatus for capturing images of elongated timber.

The apparatus 1 comprises the conveyor 40 which is arranged to convey the timber 100 transversely to a transfer direction K. The conveyor 40 comprises the conveyor upper surface 41 which is arranged to receive timber 100.

The first camera 30 is arranged relative to one or more mirror surfaces 11, 21, 51, 71 so that the first camera 30 captures images of the timber 100 only indirectly via the one or more mirror surfaces 11, 21, 51, 71 of one or more mirrors 10, 20, 50, 70 when the timber 100 is at an image capturing area 200.In one exemplary embodiment of the invention, the apparatus 1 comprises two or more first cameras 30 so that the two or more first cameras 30 being at distance from each other in a direction transverse to the transfer direction K. In other words, two or more first cameras 30 provide two or more image capturing areas 200 which are arranged to extend uniformly between the first end surface 103 of the timber 100 and the second end surface 104 of the timber 100.

For the sake of clarity, it is noted that the two or more image capturing areas 200 may be longer than the timber 100 in the direction transverse to the transfer direction K.

In one exemplary embodiment of the invention, the apparatus 1 comprises one or more light sources 300. The one or more light sources 300 are arranged to illuminate the image capturing area 200.

In other words, the one or more light sources 300 are arranged to illuminate the timber 100 on the image capturing area 200.

The embodiments shown in figure 10 may be combined with the embodiments shown in figures 1, 2a, 2b, 3, 4, 5, 6, 7, 8 and 9.

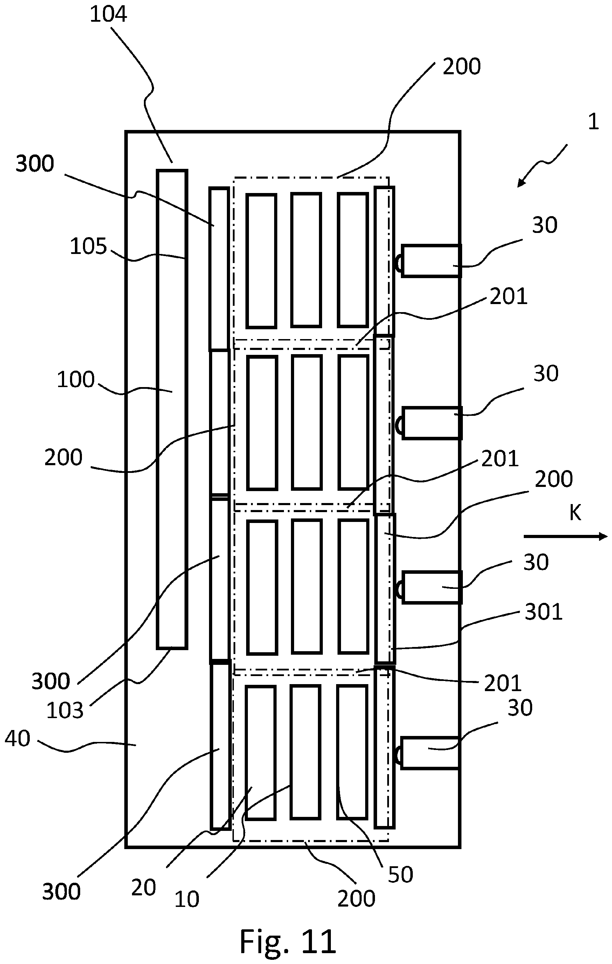

Figure 11 shows schematically a top view of an embodiment of an apparatus for capturing images of elongated timber.

In one exemplary embodiment of the invention, the apparatus 1 comprises one or more light sources 300. The one or more light sources 300 are arranged to illuminate the image capturing area 200. The one or light sources 300 being elongated.

In one exemplary embodiment of the invention, one light source 300 is arranged to illuminate two or more image capturing areas 200. The one or more light sources 300 being elongated.

In one exemplary embodiment of the invention, the apparatus 1 comprises three or more first cameras 30. Each of the first cameras 30 having an image capturing area 200. The image capturing areas 200 of adjacent first cameras 30 are arranged to form an overlapping image capturing area 201.

In other words, the image capturing areas 200 of adjacent first cameras 30 being partly overlapping.

The embodiments shown in figure 11 may be combined with the embodiments shown in figures 1, 2a, 2b, 3, 4, 5, 6, 7, 8, 9 and 10.

The invention has been described above with reference to the examples shown in the figures. However, the invention is in no way restricted to the above examples but may vary within the scope of the claims.

## Claims

1. Apparatus (1) for capturing images of elongated timber (100), **characterized in that** the apparatus (1) comprises:
- one or more mirrors (10, 20, 50, 70) having one or more mirror surfaces (11, 21, 51, 71),
- a first camera (30), and
- a conveyor (40) which is arranged to convey the timber (100) transversely to a transfer direction (K); and
the first camera (30) is arranged relative to one or more mirror surfaces (11, 21, 51, 71) so that the first camera (30) is arranged to capture images of the timber (100) only indirectly via the one or more mirror surfaces (11, 21, 51, 71).

2. Apparatus according to claim 1, **characterized in that**:
the conveyor (40) comprises a conveyor upper surface (41) which is arranged to receive timber (100); the one or more mirrors (10, 20, 50, 70) comprise a first mirror (10) and a second mirror (20); the one or more mirror surfaces (11, 21, 51, 71) comprise a first mirror surface (11) and a second mirror surface (21) so that the first mirror (10) comprises the first mirror surface (11) and the second mirror (20) comprises the second mirror surface (21); the second mirror surface (21) being transverse to the first mirror surface (11) and transverse to the conveyor upper surface (41), and the second mirror surface (21) is arranged to reflect a mirror image of the timber (100) via the first mirror surface (11) to the first camera (30).

3. Apparatus according to claim 1, **characterized in that**:
the conveyor (40) comprises a conveyor upper surface (41) which is arranged to receive timber (100); the one or more mirrors (10, 20, 50, 70) comprise a first mirror (10) and a second mirror (20); the one or more mirror surfaces (11, 21, 51, 71) comprise a first mirror surface (11) and a second mirror surface (21) so that the first mirror (10) comprises the first mirror surface (11) and the second mirror (20) comprises the second mirror surface (21); the second mirror surface (21) being transverse to the first mirror surface (11) and transverse to the conveyor upper surface (41), and the first mirror surface (11) is arranged to reflect a mirror image of the timber (100) to the first camera (30), the second mirror surface (21) is arranged to reflect a mirror image of the timber (100) to the first camera (30), and the apparatus (1) further comprises a second camera (60) aimed at the conveyor upper surface (41).

4. Apparatus according to claim 1, **characterized in that**:
the conveyor (40) comprises a conveyor upper surface (41) which is arranged to receive timber (100); the one or more mirrors (10, 20, 50, 70) comprise a first mirror (10) and a second mirror (20); the one or more mirror surfaces (11, 21, 51, 71) comprise a first mirror surface (11) and a second mirror surface (21) so that the first mirror (10) comprises the first mirror surface (11) and the second mirror (20) comprises the second mirror surface (21); the second mirror surface (21) being transverse to the first mirror surface (11) and transverse to the conveyor upper surface (41), and the second mirror surface (21) is arranged to reflect a mirror image of the timber (100) via the first mirror surface (11) to the first camera (30); the first mirror surface (11) being at a first distance (A) from the conveyor upper surface (41), the second mirror surface (21) being at a second distance (B) from the conveyor upper surface (41), and the first distance (A) being unequal to the second distance (B).

5. Apparatus according to claim 1, **characterized in that**:
the conveyor (40) comprises a conveyor upper surface (41) which is arranged to receive timber (100); the one or more mirrors (10, 20, 50, 70) comprise a first mirror (10) and a second mirror (20); the one or more mirror surfaces (11, 21, 51, 71) comprise a first mirror surface (11) and a second mirror surface (21) so that the first mirror (10) comprises the first mirror surface (11) and the second mirror (20) comprises the second mirror surface (21); the second mirror surface (21) being transverse to the first mirror surface (11) and transverse to the conveyor upper surface (41), and the second mirror surface (21) is arranged to reflect a mirror image of the timber (100) via the first mirror surface (11) to the first camera (30); the first mirror surface (11) being at a first distance (A) from the conveyor upper surface (41), the second mirror surface (21) being at a second distance (B) from the conveyor upper surface (41), and the first distance (A) being greater than the second distance (B).

6. Apparatus according to any one of claims 1 to 5, **characterized in that**:
the one or more mirrors (10, 20, 50, 70) comprise a first mirror (10), a second mirror (20) and a third mirror (50); the one or more mirror surfaces (11, 21, 51, 71) comprise a first mirror surface (11), a second mirror surface (21) and a third mirror surface (51) so that the first mirror (10) comprises the first mirror surface (11), the second mirror (20) comprises the second mirror surface (21) and the third mirror (50) comprises a third mirror surface (51).

7. Apparatus according to any one of claims 1 to 5, **characterized in that**:
the one or more mirrors (10, 20, 50, 70) comprise a first mirror (10), a second mirror (20) and a third mirror (50); the one or more mirror surfaces (11, 21, 51, 71) comprise a first mirror surface (11), a second mirror surface (21) and a third mirror surface (51) so that the first mirror (10) comprises the first mirror surface (11), the second mirror (20) comprises the second mirror surface (21) and the third mirror (50) comprises a third mirror surface (51); the third mirror surface (51) is positioned transverse to the first mirror surface (11) and transverse to the conveyor upper surface (41), the third mirror surface (51) facing in a different direction than the second mirror surface (21); and the third mirror surface (51) is arranged to reflect a mirror image of the timber (100) via the first mirror surface (11) to the first camera (30).

8. Apparatus according to any one of claims 1 to 5, **characterized in that**:
the one or more mirrors (10, 20, 50, 70) comprise a first mirror (10), a second mirror (20) and a third mirror (50); the one or more mirror surfaces (11, 21, 51, 71) comprise a first mirror surface (11), a second mirror surface (21) and a third mirror surface (51) so that the first mirror (10) comprises the first mirror surface (11), the second mirror (20) comprises the second mirror surface (21) and the third mirror (50) comprises a third mirror surface (51); the third mirror surface (51) is positioned transverse to the first mirror surface (11) and transverse to the second mirror surface (21), the first mirror surface (11), the second mirror surface (21) and the third mirror surface (51) are arranged to reflect mirror images of the timber (100) from different directions to the first camera (30).

9. Apparatus according to any one of claims 1 to 5, **characterized in that**:
the one or more mirrors (10, 20, 50, 70) comprise a first mirror (10), a second mirror (20) and a third mirror (50); the one or more mirror surfaces (11, 21, 51, 71) comprise a first mirror surface (11), a second mirror surface (21) and a third mirror surface (51) so that the first mirror (10) comprises the first mirror surface (11), the second mirror (20) comprises the second mirror surface (21) and the third mirror (50) comprises a third mirror surface (51); the third mirror surface (51) is positioned transverse to the first mirror surface (11) and transverse to the conveyor upper surface (41), the third mirror surface (51) facing in a different direction than the second mirror surface (21); and the third mirror surface (51) is arranged to reflect a mirror image of the timber (100) via the first mirror surface (11) to the first camera (30); and the third mirror (50) is arranged at a distance from the second mirror (20) in the transfer direction (K).

10. Apparatus according to any one of claims 1 to 5, **characterized in that**:
the one or more mirrors (10, 20, 50, 70) comprise a first mirror (10), a second mirror (20) and a third mirror (50); the one or more mirror surfaces (11, 21, 51, 71) comprise a first mirror surface (11), a second mirror surface (21) and a third mirror surface (51) so that the first mirror (10) comprises the first mirror surface (11), the second mirror (20) comprises the second mirror surface (21) and the third mirror (50) comprises a third mirror surface (51); the third mirror surface (51) is positioned transverse to the first mirror surface (11) and transverse to the conveyor upper surface (41), the third mirror surface (51) facing away from the second mirror surface (21), the second mirror surface (21) facing away from the third mirror surface (51), third mirror surface (51) is arranged to reflect a mirror image of the timber (100) via the first mirror surface (11) to the first camera (30); and the third mirror (50) is arranged at a distance from the second mirror (20) in the transfer direction (K).

11. Apparatus according to any one of claims 1 to 5, **characterized in that**:
the one or more mirrors (10, 20, 50, 70) comprise a first mirror (10), a second mirror (20) and a third mirror (50); the one or more mirror surfaces (11, 21, 51, 71) comprise a first mirror surface (11), a second mirror surface (21) and a third mirror surface (51) so that the first mirror (10) comprises the first mirror surface (11), the second mirror (20) comprises the second mirror surface (21) and the third mirror (50) comprises a third mirror surface (51); the third mirror surface (51) is positioned transverse to the first mirror surface (11) and transverse to the conveyor upper surface (41), the third mirror surface (51) facing towards the second mirror surface (21), the second mirror surface (21) facing towards the third mirror surface (51), third mirror surface (51) is arranged to reflect a mirror image of the timber (100) via the first mirror surface (11) to the first camera (30); and the third mirror (50) is arranged at a distance from the second mirror (20) in the transfer direction (K).

12. Apparatus according to any of claims 1 to 11, **characterized in that**:
- a distance between the first camera (30) and the conveyor upper surface (41) being less than a distance between the first mirror surface (11) and the conveyor upper surface (41); or
- a distance between the first camera (30) and the conveyor upper surface (41) being less than a distance between the first mirror surface (11) and the conveyor upper surface (41), and the first mirror surface (11) being parallel to the conveyor upper surface (41); or
- a distance between the first camera (30) and the conveyor upper surface (41) being greater than a distance between the first mirror surface (11) and the conveyor upper surface (41).

13. Apparatus according to any one of claims 1 to 11, **characterized in that**:
the one or more mirrors (10, 20, 50, 70) comprise a first mirror (10), a second mirror (20), a third mirror (50) and a fourth mirror (70); the one or more mirror surfaces (11, 21, 51, 71) comprise a first mirror surface (11), a second mirror surface (21), a third mirror surface (51) and a fourth mirror surface (71) so that the first mirror (10) comprises the first mirror surface (11), the second mirror (20) comprises the second mirror surface (21), the third mirror (50) comprises a third mirror surface (51) and the fourth mirror (70) comprises the fourth mirror surface (71); and the fourth mirror surface (71) is arranged to reflect at least a part of the first mirror surface (11) to the first camera (30); or
- the one or more mirrors (10, 20, 50, 70) comprise a first mirror (10), a second mirror (20), a third mirror (50) and a fourth mirror (70); the one or more mirror surfaces (11, 21, 51, 71) comprise a first mirror surface (11), a second mirror surface (21), a third mirror surface (51) and a fourth mirror surface (71) so that the first mirror (10) comprises the first mirror surface (11), the second mirror (20) comprises the second mirror surface (21), the third mirror (50) comprises a third mirror surface (51) and the fourth mirror (70) comprises the fourth mirror surface (71), a distance between the fourth mirror surface (71) and the conveyor upper surface (41) being less than a distance between the first mirror surface (11) and the conveyor upper surface (41), and the fourth mirror surface (71) is arranged to reflect at least a part of the first mirror surface (11) to the first camera (30).

14. Apparatus according to any of claims 1 to 13, **characterized in that**:
- the first camera (30) comprises a matrix camera; or
- the apparatus (1) comprises a second camera (60) aimed at the conveyor upper surface (41), the first camera (30) comprises a matrix camera and the second camera (60) comprises a matrix camera; or
- the first camera (30) comprises a matrix camera, and the first mirror surface (11) is arranged facing towards the conveyor upper surface (41); or
- the first camera (30) comprises a matrix camera, and the first mirror surface (11) comprises a first surface (111) and a second surface (112) so that the first surface (111) being transverse to the second surface (112); or
- the first camera (30) comprises a matrix camera, and the first mirror surface (11) comprises a first surface (111), a second surface (112) and a third surface (113) so that the first surface (111), the second surface (112) and the third surface (113) being transverse to each other.

15. Apparatus according to any one of claims 1 to 13, **characterized in that**:
- the first camera (30) is arranged above the conveyor upper surface (41); or
- the first camera (30) is arranged below the conveyor upper surface (41); or
- the first camera (30) is arranged above the conveyor upper surface (41) and the second camera (60) is arranged below the conveyor upper surface (41); or
- the first camera (30) is arranged below the conveyor upper surface (41) and the second camera (60) is arranged above the conveyor upper surface (41).
